# EUROPEAN PATENT SPECIFICATION

(11) **EP 2 684 932 B1**
(45) Date of publication and mention of the grant of the patent: **28.09.2016**
(21) Application number: 12175508.6
(22) Date of filing: 09.07.2012
(51) Int. Cl.: C09K 11/06, H05B 33/20, C07C 211/56, C07C 211/55, H05B 33/18, C07C 255/51, C07C 211/54, C07C 255/35, C07D 213/61

(54) **DIARYLAMINO MATRIX MATERIAL DOPED WITH A MESOMERIC RADIALENE COMPOUND**
DIARYLAMINO MATRIX MATERIAL DOTIERT MIT EINER MESOMEREN RADIALENVERBINDUNG
MATÉRIAU DE MATRICE DOPÉ AVEC UNE COMPOSÉ MESOMERIC RADIALÈNE

(43) Date of publication of application: 15.01.2014
(73) Proprietor: Hodogaya Chemical Co., Ltd., Tokyo 105-0011 (JP)
(72) Inventor: Werner, Ansgar, 01277 Dresden (DE); Nagaoka, Makoto, c/o Hodogaya Chemical Co., Ltd.,, Tokyo 104-0028, (JP); Kusano, Shigeru, c/o Hodogaya Chemical Co., Ltd.,, Tokyo 104-0028, (JP)
(74) Representative: Bittner, Thomas L.

(56) References cited:
- EP-A1- 2 180 029
- WO-A1-2010/145991
- WO-A1-2011/131185
- WO-A1-2011/134458
- WO-A2-2011/157385
- DE-A1-102010 004 453
- US-A1- 2010 026 176
- US-A1- 2010 102 709
- US-A1- 2010 288 362
- US-A1- 2011 315 967
- "1,4-Diazatriphenylene derivatives as hole transporting materials in electro luminescent devices", IP.COM JOURNAL, IP.COM INC., WEST HENRIETTA, NY, US, 7 June 2010 (2010-06-07), XP013138528, ISSN: 1533-0001

## Description

The present invention relates to a doped organic semiconductive matrix material and an electronic, optoelectronic or electroluminescent device comprising the doped organic semiconductive matrix material.

It has been known for several years that organic semiconductors can be heavily influenced regarding their electrical conductivity by doping. Such organic semiconductive matrix materials can be built up either from compounds with good electron donor properties or from compounds with good electron acceptor properties. Strong electron acceptors such as 2,3,5,6-tetrafluoro-tetracyano-1,4-benzoquinonedimethane (F4TCNQ) have become known for doping of electron donor materials, as described, e.g. in US2004062949, or in US2005121667. The acceptor molecules generate so-called holes by electron transfer processes in electron donor-like base materials (hole transport materials) and the conductivity of the base material is more or less significantly changed depending on the number and mobility of the holes. For example, N,N'-perarylated benzidines such as TPD or N,N',N"-perarylated starburst compounds such as the substance TDATA, or, however, also certain metal phthalocyanines, such as in particular zinc phthalocyanine ZnPc, are known as matrix material with hole transport properties.

However, the previously described compounds have disadvantages for a technical use in the production of doped semiconductive organic layers or of corresponding electronic components with such doped layers since the manufacturing processes in large-scale production plants or those on a technical scale can not always be sufficiently precise, which results in high control- and regulating expense within the processes for achieving a desired product quality or in undesired tolerances of the products. Furthermore, there are disadvantages in the use of previously known organic acceptors with regard to electronic components such as light-emitting diodes (OLEDs), field effect transistors (FET) or solar cells themselves since the cited production difficulties in the handling of the doping agents can lead to undesired irregularities in the electronic components or in undesired ageing effects of the electronic components. However, it should be considered at the same time that the doping agents to be used have extremely high electron affinities (reduction potentials) and other properties suitable for the application case since, e.g., the doping agents also co-determine the conductivity or other electrical properties of the organic semiconductive layer under given conditions. The energetic positions of the HOMO (highest occupied molecular orbital) of the matrix material and of the LUMO (lowest unoccupied molecular orbital) of the doping agent are decisive for the doping effect.

Although several different classes of p-dopants are known, it is still necessary to find out the best combinations of dopants and matrix materials for each application. Devices, such as OLEDs and organic solar cells, use hole transport materials with very deep HOMO, which require stronger dopants.

The meaning of "deep HOMO" or "low HOMO" means that HOMO is very negative taken the vacuum potential as reference. Typically, deep HOMO hole transport materials have a HOMO more negative than -5.0 eV, more preferably more negative than -5.2 eV.

The literature about doped hole transport layers refers either to the properties of the dopant or to the properties of the hole transport material. The respective other component is described in a general way with references to the state of the art. In fact, better device performance is always achieved with doped layers in comparison to the same device with non-doped layers. In this limited view it is overseen that the targeted correlated adjustment of hole transport material and dopant is necessary for the complete optimization of the device. It has to be considered that the most suitable hole transport materials for a doped layer is not necessarily the one which delivers the best performance on non-doped layers.

A main parameter for a hole transport material in a non-doped layer is the charge carrier mobility for holes. The mobility determines how much voltage is lost over this layer for a predetermined current density. In the ideal case, the mobility is so high that the voltage drop over the single layer can be neglected in view of the voltage drop over the whole device. Under this condition, the layer does not limit the current flow and the charge carrier mobility can be considered as optimized.

However this optimization level is not reached in practical situations. A considerable high voltage is required to drive hole transport layers, especially layers with colorless, transparent, hole transport materials. This is even accentuated when thicker transport layers (>50 nm) are required in the device. In those cases, the selection of the hole transport material is primarily concentrated on the maximum charge carrier mobility. Other parameters, which describe the material, are moved to the background, such as: glass transition temperature (Tg), processability, complexity and cost of production (synthesis and purification) of the material. For this reason, the high mobility material α-NPD (N,N'-Bis(naphthalene-1-yl)-N,N'-bis(phenyl)-benzidine) is the preferred hole transport material, even tough its Tg is only 96°C.

The situation is different for hole transport layers (comprising hole transport materials) doped with 3-radialene compounds. The inventors found that it is possible to achieve a lowest voltage drop for a larger number of hole transport materials. The transport layers are made conductive due to the doping effect of the 3-radialene compounds. The conductivity of the doped layers is above the threshold value of 10-5 S/cm. For such conductivity, the voltage drop over a 100 nm thick layer on a relatively high current density of 100 mA/cm2 is only 0.1 V. This value is not substantial, especially for OLEDs with an operating voltage over 3 V. In this context, it is important to realise that under those useful doped transport materials there are materials with sufficient performance, which were not used in non-doped devices because they were regarded as unsuitable. In addition, it is important to realise, that the circumstance enables new degrees of freedom for the choice of hole transport materials for doped hole transport layers.

DE102010004453A1 discloses an organic light-emitting device comprising a hole transporting layer comprising a matrix material, e. g. N,N'-Bis[(1-naphthyl)-N,N'-diphenyl]-1,1'-biphenyl)-4,4'-diamine) and a radialene dopant, namely 2,2',2"-(cyclopropane-1,2,3-trisylidene)tris(2-(p-cyanotetrafluorophenyl)acetonitrile).

US2010/0102709A1 discloses radialene compounds as well as their use as doping agent for doping an organic semiconductive matrix material, as blocker material, as charge injection layer, as electrode material as well as organic semiconductor.

WO2011/131185A1 discloses an organic semiconductor layer comprising at least one matrix material and at least one doping material.

US2010/0288362A1 discloses an organic light-emitting device comprising a matrix and radialene dopants.

The object of the invention is to find materials which deliver better conductivity in a doped hole transport layer, taking into account those materials, which would be discarded under conventional analysis.

The present invention has the object of overcoming the disadvantages of the state of the art, in particular to make new doped organic semiconductive matrix materials available that can be more readily handled in the production process, especially when preparing organic semiconductors, and that result in electronic components whose organic semiconductive materials can be reproducibly manufactured. The dopant and matrix materials shall satisfy a large number of requirements, including the correct energy levels, reasonable stability in air, high purity, thermal stability in the semiconducting layer, high processability, and so on. Also the combination of matrix and dopant shall lead to a doped semiconductor material which has a high conductivity with a low doping concentration, a high overall thermal stabiliy, and others.

This object is solved by the independent claims of the present application. Preferred embodiments are disclosed in the subclaims.

The object is achieved by a doped organic semiconductive matrix material, wherein the dopant is an organic mesomeric radialene compound with the following formula: 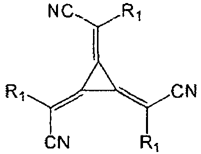 wherein each R₁ being independently selected from aryl and heteroaryl, aryl and heteroaryl being at least partially, preferably completely, substituted with electron acceptor groups;
wherein the organic semiconductive matrix material has the chemical structure (1): 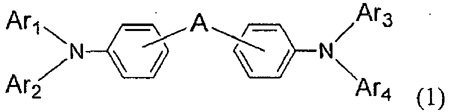 where Ar₁ to Ar₄, which may be the same or different, represent a, preferably unsubstituted, aromatic hydrocarbon group; A represents a divalent group represented by structural formulae (C) or (D) below; 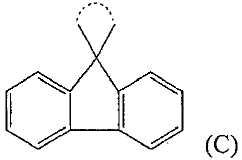 wherein the dotted line in compound (C) illustrates the bonds connecting the "A" to the two phenyl rings at the chemical structure (1); 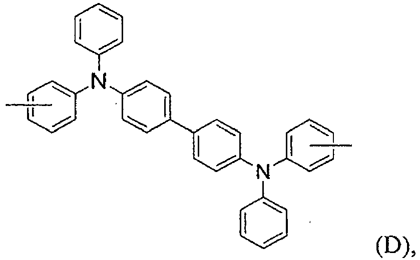 or wherein the organic semiconductive matrix material has the following structure

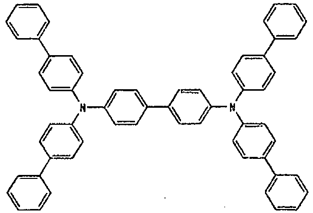

Ar₁ to Ar₄ can be, for example, phenyl or biphenyl.

The term "at least partly deuterium substituted" means that the deuterium/hydrogen ratio of the respective hydrocarbon group is higher than the natural ratio of deuterium/hydrogen.

Preferably, the matrix material has the chemical structure (1) with at least one deuterium present in structure (1).

The object is further achieved by an electronic, optoelectronic or electroluminescent device comprising a doped organic semiconductive matrix material according to the invention.

The device may be an organic light emitting device or an organic solar cell.

As a result, it was found that a better performing hole transport and 3-radialene combination is not a combination with the conventional best performing hole transport materials (those with high mobility). This will be demonstrated by the experimental examples.

The new dopants have a position of the LUMO which is so low that further technically interesting hole transport materials can now be efficiently doped for the first time. The doping effect of a certain magnitude (e.g. a doped layer of a certain conductivity) can be achieved with a substantially lower amount of dopant material to be used compared to conventional dopant under otherwise unchanged conditions. In addition, these matrix materials are extremely diffusion-stable in organic layers on account of their high polarity. Furthermore, the charge carrier injection of contacts into the doped layer can be improved by the doping agents. Furthermore, the doped organic semiconductive matrix material and a resulting electronic component can have an improved long-time stability on account of the matrix materials used in accordance with the invention. This relates to, e.g., a reduction or loss of the conductivity over time. This furthermore concerns the stability of the doped layer that is arranged adjacent to non-doped layers of an electro-optical component so that electro-optical components with increased long-time stability of the electro-optical properties such as light emission quantum yield or the like result. If the term "layer" is used throughout the description, it is understood that these layers are referred to electronic, optoelectronic or electroluminescent devices, such as organic light emitting devices or organic solar cells, which are layered devices *per se.*

By performing doping experiments, it was found that the dopant and matrix materials used in the examples below give good doping properties in its combination.

For p-doped OLED or organic solar cell, often charge transport materials such as phthalocyanine copper complex (CuPc), 4,4',4"-tris(N-3-methylphenyl-N-phenyl-amino)triphenylamine (m-MTDATA), 4,4',4"-tris[N-(2-naphthyl)-N-phenyl-amino]triphenylamine (2-TNATA) or MeO-TPD (N,N,N',N'-tetrakis(4-methoxy-phenyl)benzidine), or Spiro-TTB (2,2',7,7'-tetrakis-(N,N-diphenylamino)-9,9'-spirobifluorene, also called Spiro-TTP) are doped by acceptor materials. The layer sequence is then for instance: anode/ p-doped HIL/ EBL/ EML/ ETL/ LiF/ cathode. Herein, HIL denotes a hole injection layer, EBB denotes a electron blocking layer, EML denotes a (light) emitting layer, ETL denoted an electron transport layer, LiF denotes Lithium fluoride layer. Such HIL materials have typically a relatively low oxidation potential in the range of OV to 0.1 V vs. Fc/Fc+. Oxidation potential can be considered as a measure for the HOMO of a molecule. There is a need, however, to achieve good doping results also in host materials which are conventionally used as HTL or EBL materials. They often have a higher oxidation potential in the range of 0.2 to 0.4 V vs. Fc/Fc+. It is remarkable, that the selected dopants provide the same high conductivities in a HIL type host and a HTL type host.

### Matrix materials

The organic semiconductive matrix material is selected according to the chemical structure as disclosed above.

It has been found, that the doping of this matrix material with the selected dopant compounds provide extremely good doped charge transport layers, with low absorption, high conductivity, high thermal stability, and also a high operational stability, implying in almost no alteration of the properties during long time measurements.

### Dopants

Dopants to be used are radialene compounds with the following formula 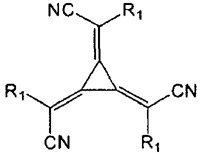 wherein each R1 being independently selected from aryl and heteroaryl, aryl and heteroaryl being at least partially, preferably completely, substituted with electron acceptor groups.

Preferred examples are the following compounds: 2,2',2"-(cyclopropane-1,2,3 -triylidene)- tris (2-perfluorophenyl-acetonitrile), 2,2',2"-(cyclopropane-1,2,3-triylidene)-tris[2-(perfluoropyridin-4-yl)-acetonitrile], 2,2',2"-(cyclopropane-1,2,3-triylidene)-tris[2-(4-cyanoperfluorophenyl)-acetonitrile], 2,2',2"-(cyclopropane-1,2,3-triylidene)-tris[2-(2,3,5,6-tetrafluoro-4-trifluoromethylphenyl)-acetonitrile], and 2,2',2"-(cyclopropane-1,2,3- triylidene)-tris[2-(2,6-dichloro-3,5-difluoro-4-trifluoromethylphenyl)-acetonitrile].

### Doping

It is understood that the compounds used in accordance with the invention are preferably such that they evaporate without any significant decomposition.

The doping agent used in accordance with the invention is preferably selected in such a manner that the semiconductive organic material doped with it still has ≥ 30%, especially preferably ≥ 60% of the conductivity (S/cm) of the initial value at room temperature (RT, 20°C) after a temperature change from RT to 100°C to RT.

The doping can take place in particular in such a manner that the molar ratio of matrix molecule to doping agent is in the range of 1:10000 (dopant molecule : matrix molecule) and 1:2, preferably between 1:100 and 1:5. An upper limit of 1:10 is preferred when high transparency is required.

### Evaporation of the doping agents

The doping of the particular matrix material (preferably indicated here as hole-conducting matrix material) with the doping agents to be used in accordance with the invention can be achieved by one or a combination of the following processes:
a) Mixed evaporation in the vacuum with a source for hole-conducting matrix material and one for the doping agent.
b) Making a solution of matrix material and dopant and form a film from the solution for instance by coating, casting or printing techniques or other film making techniques known to a person skilled in the art.

The deposition rate on a substrate with the compound used in accordance with the invention can be determined, e.g., using a quartz thickness monitor, as is customarily used, e.g., in the production of OLEDs. In particular, the ratio of the deposition rates of matrix materials and doping agent can be measured by independent measurements of them using two separate quartz thickness monitors in order to adjust the doping ratio.

### Measurement of the break down temperature

The break down temperature can be measured with a method in which the (undoped or doped) layer is heated stepwise, and after a delay the conductivity is measured. The maximum temperature, which can be applied to the layer without loosing the desired semiconducting properties is then the temperature just before the conductivity breaks down. For example, a doped layer can be heated on the substrate with two electrodes, as disclosed above, in steps of 1°C, wherein after each step there is a waiting period of 10 seconds. Then the conductivity is measured. The conductivity changes with temperature and breaks down abruptly at a particular temperature. The temperature stability is therefore the maximum temperature up to which the conductivity does not break down abruptly. The measurement is performed in vacuum.

### Electronic component

A plurality of electronic components or equipment containing them can be produced using the organic compounds in accordance with the invention for producing doped organic semiconductive materials that can be arranged in particular in the form of layers or electrical line paths. In particular, the doping agents used in accordance with the invention can be used to produce organic light-emitting diodes (OLED), organic solar cells, organic diodes, especially those with a high rectification ratio such as 10³-10⁷, preferably 1.0⁴-10⁷ or 10⁵-10⁷ or organic field effect transistors. The conductivity of the doped layers and/or the improvement of the charge carrier injection of contacts into the doped layer can be improved by the doping agents in accordance with the invention. In particular in the case of OLEDs or solar cells the component can have a pin structure (the device has one or more p-doped hole transport layers and/or one or more n-doped electron transport layers) or an inverted structure (the top-electrode and hole transport layer are located on the same side from the light emitting or light harvesting layer while the substrate is on the opposite side) without being limited to them. An injection layer can be made, for instance, by forming a layer containing or consisting of the organic compounds in accordance with the invention between an electrode and a charge transporting layer. However, the use of the doping agents in accordance with the invention is not limited to the advantageous exemplary embodiments cited above.

### Exemplary embodiments

The invention will be explained in detail on the basis of exemplary embodiments.

The compounds will now be used in the following manner as doping agents for different hole conductors that for their part are used for constructing certain microelectronic or optoelectronic components such as, e.g., an OLED. The doping agents can be co-evaporated with the hole transport materials of the matrix in high vacuum (ca. 2 x 10⁻⁴Pa) by thermal evaporation. A typical deposition rate for the matrix material is 0.2 nm/s (density ca. 1.5 g/cm³). The evaporation rates for the doping agents can vary between 0.001 and 0.5 nm/s (assuming the same density) in accordance with the desired doping ratio.

In the following examples the current measurements were carried out over a current path of the doped hole-conducting matrix material 1 mm long and ca. 0.5 mm wide at 1V. Under these conditions compound-1 (see below) conducts practically no electrical current.

### Synthesis of dopants:

### 1. Ethyl 2-cyano-2-aryl acetates a), b), c), d) and e)

### General procedure:

To a solution of 207 mmol of the either starting material A, B, C, D or E and 250 mmol of potassium carbonate in 370 ml of dimethylformamide, 207 mmol of cyano acetic ester in 50 ml of dimethylformamid were added quickly. The mixture was allowed to stirr for 48 h at room temperature. Then the reaction suspension was poured into a 3L beaker with 1L of ice water. While stirring the solution was acidified with 100mL of conc. acetic acid. The aqueous solution was extracted four times with chloroform in this order (250mL, 150mL, 100mL, 100mL). After drying the combined organic layers with magnesium sulphate the solvent was removed in vacuum. The remaining oil was used in the next step without any further purification.

| Starting material | Corresponding acetate |
|---|---|
| Hexafluorobenzene (A) | (a) Ethyl 2-cyano-2-(perfluorophenyl) acetate |
| Pentafluoropyridin (B) | (b) Ethyl 2-cyano-2-(perfluoropyridin-4-yl) acetate |
| Pentafluorobenzonitril (C) | (c) Ethyl 2-cyano-2-(4-cyanoperfluorophenyl) acetate |
| Octafluorotoluene (D) | (d) Ethyl 2-cyano-2-(4-trifluoromethylperfluorophenyl) acetate |
| 4-trifluoromethyl-2,6-dichloro-1,3,5-trifluorobenzene (E) | (e) Ethyl 2-cyano-2-(4-trifluoromethyl-2,6-dichloro-3,5-difluorophenyl) acetate |

### 2. Aryl acetonitriles (f), (g), (h), (i) and (k):

### General procedure:

In a 250mL round bottom flask the whole amount of the ethyl 2-cyano-2-aryl acetate (a), (b), (c), (d), or (e) as synthesized above was dissolved in 84 ml of acetic acid (50%) together with 4.15 ml of sulfuric acid (conc.). The mixture was heated on reflux for 16 hours. After cooling to room temperature the mixture was poured into a 500mL beaker with 120mL of ice water and stirred over a period of 30 min. The organic layer was separated and the aqueous layer extracted with 100mL of chloroform. The combined organic layers were washed with 100mL of water and with 100mL of saturated sodium bicarbonate solution. After drying the organic layer with magnesium sulphate the solvent was removed in vacuum to give brown coloured oil. Distillation in vacuum gave a colourless slow solidifying liquid.

| acetate | Intermediate compounds, aryl acetonitriles |
|---|---|
| (a) | (f) Pentafluorophenyl acetonitrile |
| (b) | (g) 4-(cyanomethyl)-2,3,5,6-tetrafluoropyridine |
| (c) | (h) 4-(cyanomethyl)-2,3,5,6-tetrafluorobenzonitril |
| (d) | (i) 2-(2,3,5,6-tetrafluoro-4-trifluoromethylphenyl)acetonitrile |
| (e) | (k) (4-trifluoromethyl-2,6-dichloro-3,5-difluorophenyl)acetonitrile |

### 3. [3]-Radialenes (1), (m), (n), (o), and (p)

### General procedure:

Lithium hydride (98%) is suspended in 600mL of 1,2- dimethoxyethane and cooled to 0°C. 152 mmol of aryl acetonitrile (f), (g), (h), (i), or (k) was dissolved in 60mL of 1,2-dimethoxyethane and added over a period of 10 to 15 min. The ice bath has been removed and the reaction was allowed to warm up over 45 min. After 15 min stirring at room temperature the mixture was cooled to 0°C again. 7.12 g (40.0 mmol) of perchlorocycloprop-1-ene in 40mL of 1,2-dimethoxyethane were added dropwise. The colour of the solution turned to dark red. The dark solution was kept on stirring for 44 h while warming up to room temperature. Then the reaction suspension was poured into a 2 L beaker with 1.2 L of ice water. The stirring solution was acidified with concentrated hydrochloric acid to pH=1 (240 mL HCl) and extracted with ethyl acetate (3x500 mL). The combined organic layers were washed in the following order with brine, water and bicarbonate solution and then with water again. The combined organic layers were dried with magnesium sulphate and the solvent was carefully removed in vacuum to give a dark coloured material which was directly used in the next transformation without any further purification.

The dark coloured material was dissolved in 1400mL of acetic acid (100%) and treated with a mixture of 360mL hydrobromic acid (48%) and 120mL of nitric acid (65%) prepared approximately ten minutes before. The resulting mixture was stirred for 1.5 hours. The mixture was filtrated and the resulting orange precipitate was washed with water and dried in vacuum to afford the crude material. The crude material was purified by gradient sublimation.

| acetate | Final compound, [3]-radialenes |
|---|---|
| (f) | (1) 2,2',2"-(cyclopropane-1,2,3 -triylidene)-tris(2-perfluorophenylacetonitrile) |
| (g) | (m) 2,2',2"-(cyclopropane-1,2,3-triylidene)-tris[2-(perfluoropyridin-4-yl)-acetonitrile] |
| (h) | (n) 2,2',2"-(cyclopropane-1,2,3-triylidene)-tris[2-(4-cyanoperfluorophenyl)-acetonitrile] |
| (i) | (o) 2,2',2"-(cyclopropane-1,2,3-triylidene)-tris[2-(2,3,5,6-tetrafluoro-4-trifluoromethylphenyl)-acetonitrile] |
| (k) | (p) 2,2',2"-(cyclopropane-1,2,3-triylidene)-tris[2-(2,6-dichloro-3,5-difluoro-4-trifluoromethylphenyl)-acetonitrile] |

### Synthesis of matrix materials:

### Synthesis Example 1, Synthesis of Compound-1

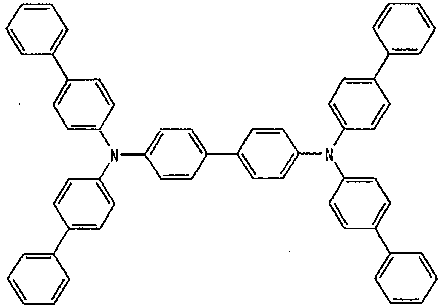

A mixture of bis(biphenyl-4-yl)amine 10.0g(31.1mmol), 4,4'-Diiodobiphenyl 5.28g (30.0mmol), anhydrous potassium carbonate 5.39g(39.0mmol), copper powder 0.083g (1.3mmol), dodecylbenzene 12ml and xylene 24ml was stirred and heated. After xylene was evaporated the mixture was reacted at 210 - 215°C for 4 hours. Furthermore copper powder 0.083g(1.3mmol) was added to the mixture, additional reaction was proceeded for 4.5 hours. After cooling to 90°C, toluene was added to the reaction mixture, and the reaction mixture was cooled to room temperature. The reaction mixture was filtered and washed with water, methanol and toluene. The residue was extracted with hot toluene and toluene solution was concentrated to dry-up in vacuo. The crude product obtained was purified by recrystallization. 7.38g (yield:71.7%) of N,N,N',N'- Tetrakis (biphenyl-4-yl)-4,4'-diaminobiphenyl was obtained. The melting point was 265°C.

### Synthesis Example 2, Synthesis of Compound-2

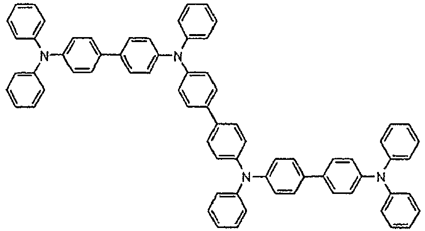

Acetanilide 20.3g(0.15mol), 4,4'-diiodobiphenyl 73.1g(0.18mol), anhydrous potassium carbonate 22.1g(0.16mol), copper powder 2.16g(0.034mol) and nitrobenzene 35ml were mixed. The reaction mixture was stirred for 10 hours at 190 - 205°C. The reaction mixture was extracted with toluene 200ml. The insoluble matters were removed by filtration. The filtrate was concentrated to dry-up. The crude product obtained was purified by column chromatography (carrier was silica gel and eluent was 6/1 mixture of toluene and ethyl acetate). 40.2g (yield: 64.8%) of N-(4'-iodo-4-biphenylyl)acetanilide was obtained. A melting point of the product was 135.0 - 136.0°C.

Subsequently, N-(4'-iodo-4-biphenylyl)acetanilide 13.2g(0.032mol), diphenylamine 6.60g(0.039mol), anhydrous potassium carbonate 5.53g(0.040mol), copper powder 0.45g(0.007 mol) and nitrobenzene 10ml were mixed. The reaction mixture was stirred for 15 hours at 200 - 212!C. The reaction mixture was extracted with toluene 100ml. The insoluble matters were removed by filtration. The filtrate was concentrated to dry-up. An oily matter was obtained. The oily matter was dissolved in isoamyl alcohol 60ml. The material was hydrolyzed with water 1ml and 85% potassium hydroxide 2.64g(0.040mol) at 130°C. The reaction solution was then distilled by steam distillation to remove isoamyl alcohol. The residue was extracted with toluene 250ml, toluene solution was washed with water and concentrated to dry-up. The crude product obtained was purified by column chromatography (carrier was silica gel and eluent was 1/2 mixture of toluene and n-hexane). 10.5g (yield: 72.2%) of N,N,N'-triphenyl-4,4'-diaminobiphenyl was obtained. The compound melted at 167.5 - 168.5°C.

N,N,N'-'Triphenyl-4,4'-diaminobiphenyl 8.66g(0.021mol), 4,4'-diiodobiphenyl 4.06g(0.01mol), anhydrous potassium carbonate 2.90g(0.021mol), copper powder 0.32g(0.005mol) and nitrobenzene 10ml were mixed. The reaction mixture was stirred at 195 - 210°C for 20 hours. The reaction mixture was extracted with toluene 140 ml. The insoluble matters were removed by filtration. The filtrate was concentrated and a crude product was obtained. n-Hexane 120ml was added to the crude product to recover a crude crystal. The crude crystal was purified by column chromatography (carrier was silica gel and eluent was 1/2 mixture of toluene and n-hexane). 4.73g (yield: 48.5%) of N,N'-Bis[4'-(diphenylamino) biphenyl-4-yl]-N,N'-diphenyl-4,4'-diaminobiphenyl was obtained. The melting point of the product was 242.5 - 243.5°C.

The result of elementary analysis on the product was as follows.
The theoretical value : (carbon 88.67%) (hydrogen 5.58%) (nitrogen 5.75%)
Actual measurement value : (carbon 88.75%) (hydrogen 5.70%) (nitrogen 5.68%)

### Synthesis Example 3, Synthesis of Compound-3

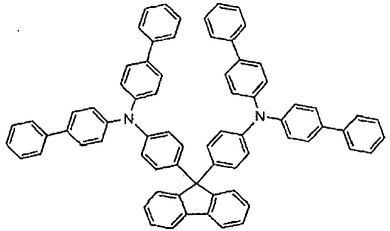

A mixture of bis(biphenyl-4-yl)amine 20.30g(63.16mmol), 9,9-his(4-iodophenyl)fluorene 15.0g(26.31mmol), anhydrous potassium carbonate 10.91g(78.92mmol), copper powder 0.17g (2.63mmol) and dodecylbenzene 30ml was stirred and heated at 200°C for 13 hours under nitrogen gas.

The reacting mixture was analyzed by HPLC, it was confirmed that the monoiodo-compound of intermediates was 1% or less, and assumed a reactive end.

Toluene 100ml was added to the reaction mixture and filtered. A residue was washed with methanol and water.

The residue was added in chlorobenzene 2000ml and heated to 120°C. After cooling to 60°C the mixture was filtered and the filtrate was concentrated to dry up. The crude product obtained was purified by recrystallization by chlorobenzene for 3 times.

The purified product, 4',4"-bis[bis(4-biphenylyl)amino]-9,9-diphenylfluorene was 17.9g of gray-white powder. Yield was 71% and melting point was 324 - 326°C.

### Synthesis Example 4, Synthesis of Compound-4

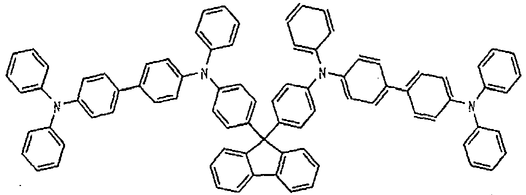

4',4"-Bis[(4'-phenylaminobiphenyl-4-yl)amino]-9,9-diphenylfluorene 8.4g(10mmol), iodobenzene 10.2g(50mmol), anhydrous potassium carbonate 8.3g(60mmol), copper powder 0.64g(10mmol), tridecane 100ml were mixed and reacted for 10 hours at reflux temperature under nitrogen gas. The reaction mixture was extracted with toluene 120ml and insoluble matters were removed with filtration. The filtrate was concentrated to dry up and crude product was obtained. The crude product was purified by column chromatography with silica gel. The purified product, 4',4"-Bis[N-(4'-diphenylaminobiphenyl-4-yl)-N-phenyl-amino]-9,9-diphenylfluorene was 5.4g of white powder. Yield was 47% and melting point was 186 - 220°C.

The chemical structure of the obtained white powder was identified with 13C-NMR. In 13C-NMR, 24 peaks among 26 peaks that correspond to aromatic 84 carbon atoms were 120.03, 122. 71, 123.20, 123.97, 124.13, 124.21, 124.41, 125.20, 126.14, 127.16, 127.29, 127.51, 128.12, 128.83, 128.92, 129.13, 134.62, 139.77, 139.94, 145.91, 146.45, 146.58, 147.58, 151.40 ppm and a peak of aliphatic carbon atom in fluorene 9-position was 64.56 ppm.

In addition, the result of elementary analysis on the obtained white powder was as follows.
The theoretical value : (carbon 89.6%) (hydrogen 5.5%) (nitrogen 4.9%)
Actual measurement value : (carbon 89.5%) (hydrogen 5.6%) (nitrogen 4.4%)

The chemical structure of compound 4 was identified from the results of 13C-NMR and the elementary analysis.

### Doping Examples:

### Example 1:

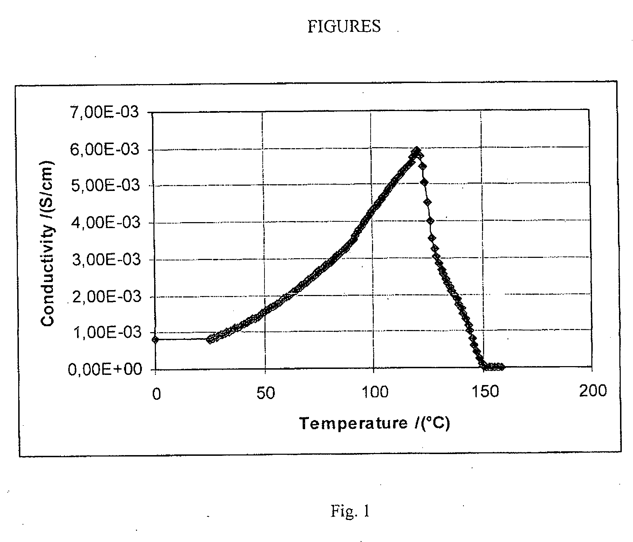

A mixed layer of compound-1 as a matrix material and compound (h) as a dopant material have been made by mixed thermal evaporation on a glass substrate in a high vacuum chamber. The doping concentration was 5mol%, the film thickness 50nm. The glass substrate has two ITO stripes with a distance of 1mm as electrodes for the film. From the current-voltage characteristics of the film and the geometry of the sample, the conductivity of the mixed layer was determined to be 8.20·10⁻⁴ S/cm. Figure 1 shows the evolution of the conductivity of a planar device made consisting of two laterally spaced electrodes and a doped layer of compound-1 and compound (h). The device was heated at an approximate rate of 1 °C/minute and the conductivity was measured. It can be seen that the conductivity breaks down at a temperature of 121 °C, which temperature defines Tstab.

### Example 2:

Another film has been made like in example 1. As the dopant, compound (k) was used. The conductivity of the film was 4.17·10⁻⁴ S/cm.

Conductivities above 10⁻⁵ S/cm are considered sufficient for doped semiconducting charge carrier transport materials to be used in OLEDs.

Several conductivity measurements were made with matrix materials according to the invention. Comparison of the conductivity with different dopants shows that the selected dopants lead to much higher conductivities (10 mol% dopant concentration).

| Material | Eox (V vs. Fc/Fc⁺) | Conductivity with F4-TCNQ / S/cm x 10⁻⁶ | Conductivity with compound (h)* / S/cm x 10⁻⁶ | Conductivity with compound (k)* / S/cm x 10⁻⁶ |
|---|---|---|---|---|
| HTM-1 | 0.31 | 0.6 | 80 (92) | 40 |
| HTM-2 | 0.10 | 16 | 324 (92) | 338 |
| Compound-1 | 0.28 | 0.02 | 820 (121) | 183 |
| Compound-2 | 0.24 | 0.3 | 3000 | 735 (112) |
| Compound-3 | 0.44 | 0.04 | 130(96) | 69 |
| Compound-4 | 0.29 | 2 | 120 (98) | 62 |
| * values in parenthesis: breakdown temperature of conductivity | | | | |

The features of the invention disclosed in the previous description, in the claims and the drawing can be essential individually as well as in any combination for the realization of the invention in its various embodiments.

### Part list

- HIL: - Hole injection layer
- HTL: - Hole transport layer
- EBL: - Electron blocking layer
- EML: - Emiter layer
- ETL: - Electron transport layer
- HOMO: - Highest occupied molecular orbital
- LUMO: - Lowest unnocuupied molecular orbital
- p-i-n: - p-doped HTL - non-doped layer(s) - n-doped ETL
- HTM-1: - N,N'-Bis(naphthalene-1-yl)-N,N'-diphenylbenzidine
- HTM-2: - 2,2',7,7'-tetrakis[N,N-bis(p-methylphenyl)amino]-9,9'-spirobifluorene

## Claims

1. Doped organic semiconductive matrix material, **characterized in that** the dopant is an organic mesomeric radialene compound with the following formula: 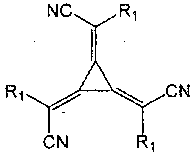 wherein each R₁ being independently selected from aryl and heteroaryl, aryl and heteroaryl being at least partially, preferably completely, substituted with electron acceptor groups;
wherein the organic semiconductive matrix material has the chemical structure (1): 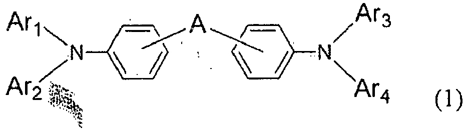 where Ar₁ to Ar₄, which may be the same or different, represent a, preferably unsubstituted, aromatic hydrocarbon group; A represents a divalent group represented by structural formulae (C) or (D) below; 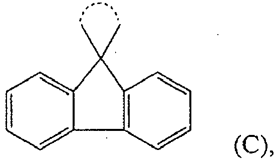 wherein the dotted line in compound (C) illustrates the bonds connecting the "A" to the two phenyl rings at the chemical structure (1); 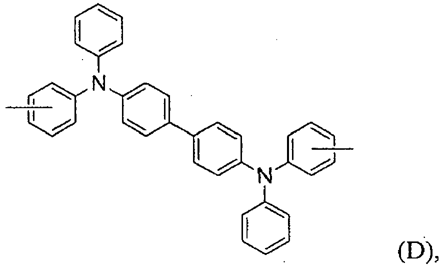 or wherein the organic semiconductive matrix material has the following structure

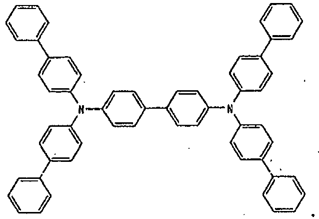

2. Matrix material according to claim 1, wherein the matrix material has the chemical structure (1) with at least one deuterium present in structure (1).

3. Electronic, optoelectronic or electroluminescent device comprising a doped organic semiconductive matrix material according to any of the preceding claims.

4. Electronic, optoelectronic or electroluminescent device according to claim 3, wherein the device is an organic light emitting device or an organic solar cell.

## Patentansprüche

1. Dotiertes organisches halbleitendes Matrixmaterial, **dadurch gekennzeichnet, dass** der Dotand eine organische mesomere Radialenverbindung der folgenden Formel ist: 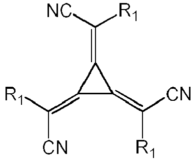 wobei jedes R₁ unabhängig voneinander ausgewählt ist aus Aryl und Heteroaryl, wobei Aryl und Heteroaryl zumindest teilweise, vorzugsweise vollständig, mit Elektronenakzeptorgruppen substituiert sind;
wobei das organische halbleitende Matrixmaterial die chemische Struktur (1) hat: 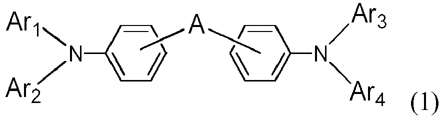 wobei Ar₁ bis Ar₄, die gleich oder unterschiedlich sein können, eine, vorzugsweise unsubstituierte, aromatische Kohlenwasserstoffgruppe darstellen; A eine divalente Gruppe darstellt, die dargestellt ist durch die Strukturformeln (C) oder (D), wie im Nachfolgenden angegeben; 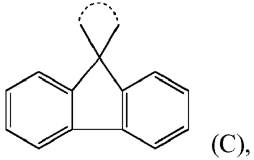 wobei die gepunktete Linie in der Verbindung (C) die Bindungen darstellen, mit denen das "A" mit den beiden Phenylringen der chemischen Struktur (1) verbunden ist; 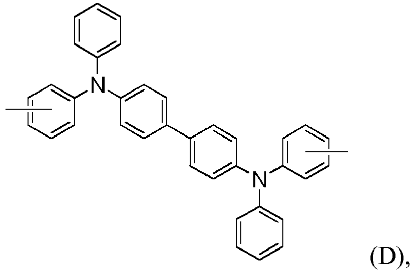 oder wobei das organische halbleitende Matrixmaterial die folgende Struktur hat

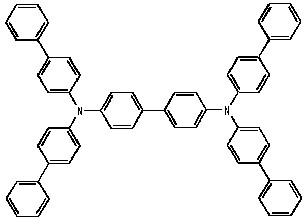

2. Matrixmaterial nach Anspruch 1, wobei das Matrixmaterial die chemische Struktur (1) hat, wobei zumindest ein Deuterium in der Struktur (1) vorhanden ist.

3. Elektronische, optoelektronische oder elektrolumineszente Vorrichtung, umfassend ein dotiertes organisches halbleitendes Matrixmaterial nach einem der vorangehenden Ansprüche.

4. Elektronische, optoelektronische oder elektrolumineszente Vorrichtung nach Anspruch 3, wobei die Vorrichtung eine organische lichtemittierende Vorrichtung oder eine organische Solarzelle ist.

## Revendications

1. Matériau de matrice semi-conducteur organique dopé, **caractérise en ce que** le dopant est un composé radialène mésomère organique de la formule suivante : 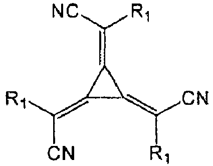 dans laquelle chaque groupe R₁ étant indépendamment choisi parmi aryle et hétéroaryle, aryle et hétéroaryle étant au moins partiellement, de préférence complétement, substitués par des groupes accepteurs d'électrons ;
dans laquelle le matériau de matrice semi-conducteur organique possède la structure chimique (1) : 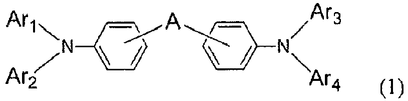 où Ar₁ à Ar₄, qui peuvent être identiques ou différents, représentent, de préférence, un groupe hydrocarboné aromatique, non substitué ; A représente un groupe divalent représenté par les formules structurales (C) ou (D) ci-dessous ; 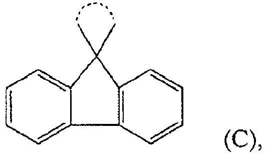 dans laquelle la ligne pointillée dans le composé (C) illustre les liaisons connectant les « A » aux deux noyaux phényle à la structure chimique (1) ; 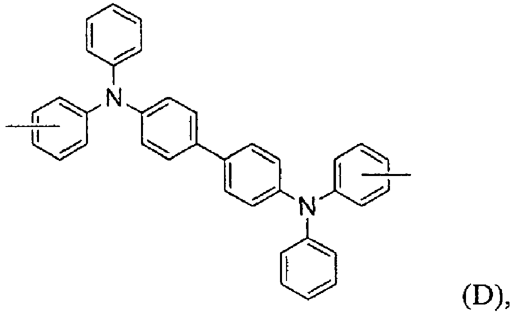 ou dans laquelle le matériau de matrice semi-conducteur organique possède la structure suivante

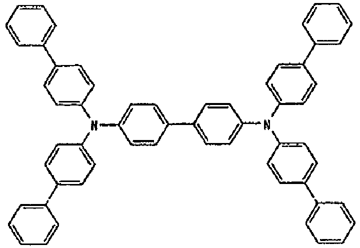

2. Matériau de matrice selon la revendication 1, dans lequel le matériau de matrice possède la structure chimique (1) avec au moins un deutérium présent dans la structure (1).

3. Dispositif électronique, optoélectronique ou électroluminescent comprenant un matériau de matrice semi-conducteur organique dopé selon l'une des revendications précédentes.

4. Dispositif électronique, optoélectronique ou électroluminescent selon la revendication 3, dans lequel le dispositif est un dispositif organique émettant de la lumière ou une cellule solaire organique.
